# EUROPEAN PATENT SPECIFICATION

(11) **EP 4 186 448 B1**
(45) Date of publication and mention of the grant of the patent: **24.07.2024**
(21) Application number: 21210488.9
(22) Date of filing: 25.11.2021
(51) Int. Cl.: A61B 17/34

(54) **A TROCAR FIXATION ASSEMBLY**
TROKARFIXIERUNGSANORDNUNG
ENSEMBLE DE FIXATION DE TROCART

(43) Date of publication of application: 31.05.2023
(73) Proprietor: Mölnlycke Health Care AB, 402 52 Göteborg (SE)
(72) Inventor: ROSENGREN, Oscar, HOVÅS (SE)
(74) Representative: Kransell & Wennborg KB

(56) References cited:
- EP-A1- 2 965 700
- EP-A2- 1 402 821
- WO-A1-2016/186937
- US-A- 3 021 842
- US-A1- 2021 068 917

## Description

### TECHNICAL FIELD

The present disclosure generally relates to a trocar fixation assembly comprising a fixation device and a medical dressing. The present disclosure also relates to a trocar system comprising the trocar fixation assembly.

### BACKGROUND

Minimal invasive surgery (MIS) is a technique for performing surgery that is associated with less pain, shorter recovery and fewer complications compared to traditional open surgery.

In an MIS procedure, a small incision is typically made through the skin of a patient, and a trocar is thereafter inserted into the incision. The trocar serves as a working channel that allows for the entry of various surgical instruments and cameras into a body cavity, e.g. an abdominal cavity.

A trocar typically comprises a cannula and an obturator. The cannula is a hollow and tube-shaped shaft that is placed inside the patient to provide access to the body cavity during the MIS procedure. The obturator is a sharp tool that allows the cannula to penetrate the body cavity for initial placement. When the cannula has entered the body cavity, the obturator is removed.

During an MIS procedure, a plurality of trocars is typically utilized. Accordingly, the surgeon can utilize a variety of surgical instruments and tools and alternate between the different trocars throughout the procedure. Often, the surgeon utilizes more than one surgical tool at the same time. In such scenarios, the medical staff must typically support the surgeon in fixating the trocar, and in facilitating the access to the various trocars.

In many situations, the trocar needs to be inserted in a tilted configuration in the body cavity. In order to keep a trocar in a tilted configuration, a member of the surgical staff may need to hold the trocar in position. If the trocar is allowed to move freely within the body cavity and is repeatedly exposed to dislocation, this may cause undesired movement of the trocar inside the patient, and also increase the tension of the surgical incision and the skin area surrounding the incision. Furthermore, the surgeon must typically lean over the patient in order to access the plurality of trocars inserted into the skin of the patient. This may be challenging from an ergonomic perspective.

Another challenge during MIS procedures is sterility and keeping the surgical sites and the areas circumventing the trocars clean and free from blood and contaminants.

US 2021/068917 A1, WO 2016/186937 A1 and EP 1402821 A2 disclose examples of fixation assemblies to stabilize surgical tools, the assembly comprising at least two threaded annular members housing a rotatable ball member, and an adhesive layer to fix the assembly to a surgical site.

In view of this, it would be desirable to facilitate the access to the trocars during the MIS procedure while also preventing dislocation of the trocar cannulas after insertion into the body cavity. In addition, it would be desirable to improve the sterility during the MIS procedure.

Accordingly, there is a need to provide an improved and simplified means for fixating a trocar while also relieving the burden for the surgical staff in performing the minimal invasive surgery. Such means should provide for a sterile, simplified, and safe MIS procedure to be carried out.

### SUMMARY

In view of the above-mentioned problems, it is an object of the present disclosure to provide improvements with respect to relieving the burden for surgical staff and providing a facilitated and improved means to handle and fixate a trocar during minimal invasive surgery. The invention is defined by independent claim 1, while further embodiments are defined by the dependent claims.

According to a first aspect, there is provided a trocar fixation assembly comprising a fixation device and a medical dressing, wherein the fixation device comprises:
- a skin attachment element comprising a first annular member disposed about a longitudinal center line,
- a ball member arranged in the first annular member, wherein the ball member comprises a channel extending through the ball member and being configured to receive a trocar, and
- a second annular member disposed about the longitudinal center line and being configured to engage with the first annular member,
wherein the engagement between the first and second annular members allows for the trocar fixation assembly to be shifted between at least a first configuration, in which the ball member is movable with respect to the first and second annular members, a second configuration, in which the ball member is gradually movable with respect to the first and second annular members, and a third configuration, in which the ball member is fixed with respect to the first and second annular members.

The present disclosure is based on the realization that a trocar fixation assembly as defined hereinabove greatly facilitates the minimally invasive surgical procedure for the surgeon and for the surgical staff.

The trocar fixation assembly is configured to be shifted between a first, a second and a third configuration. In the first configuration, the ball member is freely moveable with respect to the first and the second annular members. A trocar inserted into the channel of the ball member may thus pivot 360 degrees about the longitudinal center line of the first and second annular members.

The movable, first configuration allows the surgeon to adjust the angular position of the trocar depending on his/her position in the room and depending on the specific surgical situation. Furthermore, the first, movable configuration allows the surgeon to change the position of the trocar in a simplified and more ergonomic manner. The surgeon does not need to lean over the patient or change position in the room in order to operate through the trocars, e.g. by inserting a variety of surgical tools into the trocar cannulas.

In the second configuration, the ball member is gradually movable with respect to the first and second annular members. Accordingly, the ball member may be moved, but the movement or pivoting thereof requires a larger force. In the absence of a "pushing force"; i.e. in the absence of moving or adjusting the trocar, the ball member may be fixed in position. This allows for the surgeon to leave a trocar in the trocar fixation assembly and proceed with a second trocar (inserted into a second trocar fixation assembly) utilizing a different surgical tool. When the surgeon or surgical staff wishes to continue the operation through the first trocar fixation assembly, the surgeon may grasp or move the trocar, which allows for the ball member to be moved, albeit in a slower manner. The position of the trocar may thus be adjusted by moving the trocar gradually, and in a significantly gentler manner within the body cavity.

This is beneficial since it prevents undesired movement of a trocar inside a body cavity of the patient (which may be risky), but still allows the surgeon to adjust the position of the trocar (and ball member) in a situation specific manner.

In the third configuration, the ball member is fixed with respect to the first and second annular members.

In instances where it is desired for the trocar to be completely fixed in position, the trocar fixation assembly may be shifted to the third configuration. In this configuration, the ball member is prevented from movement in any direction.

The trocar fixation assembly has a simple construction and greatly facilitates the MIS procedure for the surgical staff, while also improving the patient safety.

In exemplary embodiments, each of the first and the second annular members comprises interior walls, and exterior walls, wherein at least a portion of the exterior walls of the first annular member is threaded, and wherein at least a portion of the interior walls of the second annular member is threaded, wherein the first and second annular members are configured to be threadedly engaged.

Accordingly, the trocar fixation assembly may be shifted between the first, second and third configuration by means of a screw mechanism. A simple and easily practicable trocar fixation assembly is thereby provided.

The ball member is disposed about a longitudinal axis, and wherein the ball member may be shifted between a straight configuration, in which the longitudinal axis of the ball member coincides with the longitudinal center line, and a slanted configuration, in which the longitudinal axis of the ball member is arranged at an angle, α, with respect to the longitudinal center line.

In some scenarios, it may be desired to fix the ball member in a straight manner with respect to an incision. In other scenarios, it is desired to fix or move the ball member in the slanted configuration. The slanted configuration allows for the surgeon to access and reach the trocar in a simplified manner, regardless of his/her position in the room.

In exemplary embodiments, the longitudinal axis of the ball member is arranged at an angle, α, of from 5 to 60°, preferably from 10 to 45° with respect to the longitudinal center line in the slanted configuration.

The angle, α, between the longitudinal axis and the longitudinal center line is preferably in the above specified ranges to optimize the handling of a trocar. If the angle, α, is too high, the risk of harmful intervention in the body cavity is increased. If the angle, α, is too low, the surgeon may face difficulties in grasping and reaching the trocar.

In exemplary embodiments, the first annular member defines a first aperture having a diameter, d1; the second annular member defining a second aperture having a diameter, d2, wherein the diameter, d3, of the channel of the ball member is smaller than the diameter, d1, of the first aperture and the diameter, d2, of the second aperture.

The diameter, d3, of the channel is dimensioned to receive a trocar cannula. Accordingly, the diameter, d3, substantially corresponds to the diameter of the trocar cannula.

The diameters, d1, and d2 of the first and the second apertures of the first, and the second annular member, respectively are larger than the diameter, d3, of the channel of the ball member.

The diameter, d1, of the first aperture of the first annular member is selected to allow the ball member to reside in the annular member and to be movable and rotatable therein. The diameter, d2, of the second aperture of the second annular member is selected to allow for the second annular member to engage with the first annular member and also to allow for the ball member to move with respect to the second annular member.

In exemplary embodiments, at least a portion of the channel of the ball member comprises a plurality of annual grooves.

The grooves may be arranged along the perimeter of the interior walls of the channel.

Accordingly, a trocar cannula comprising threads, ribs or ridges may engage with the grooved channel of the ball member by means of a threading mechanism. Hence, the trocar may be fixed in position along the longitudinal axis of the ball member and is prevented from undesired projection into and out of the body cavity. In cases where the surgeon wishes to penetrate deeper into the body cavity, the trocar may be threadedly moved by screwing the trocar.

In exemplary embodiments, the interior walls, and exterior walls, of the first annular member extend from a bottom surface to a top surface, and wherein the skin attachment element comprises a base member surrounding the bottom surface of the first annular member.

The base member is arranged to contact the skin of the patient, i.e. the skin circumventing the incision.

In exemplary embodiments, the medical dressing comprises a centrally disposed aperture configured to encircle the first annular member.

The medical dressing typically encircles the first annular member such that essentially no gaps are formed between the fixation device and the dressing. This is to avoid potential entry of contaminants.

The medical dressing may be arranged to overlap the base member of the skin-attachment element. Accordingly, the parts of the dressing extending beyond the contour of the base member of the skin attachment assembly may be adhesively attached to the skin. Furthermore, the provision of the base member secures that no gaps between the annular member and the dressing are formed.

In exemplary embodiments, the medical dressing is arranged between the base member and the second annular member.

Accordingly, the medical dressing may be clamped between the base member and the second annular member during use. The medical dressing may be detachably or fixedly attached to the base member.

During use, the medical dressing is typically fixed between the base member and the second annular member. After surgery, when the trocar is to be exerted from the body cavity and the trocar fixation assembly is to be removed, each of the components may be removed separately, or together, if desired.

In exemplary embodiments, the medical dressing extends uninterrupted around the first annular member.

A tight seal is thereby provided, and the sterility is improved in the area circumventing the surgical site since the dressing contains no cuts or openable parts.

In exemplary embodiments, the medical dressing comprises a backing layer and an adhesive skin contact layer.

The adhesive skin contact layer secures a tight fit to the skin, and the dressing is fixed in place in the area circumventing the surgical site (and the fixation device). Blood and body fluids exuded from the surgical site may be evaporated through the backing layer.

In exemplary embodiments, the adhesive skin contact layer comprises a silicone based adhesive.

A silicone based adhesive is skin-friendly and gentle to the skin. The silicone based adhesive allows for the dressing to be removed in a gentle manner from the skin without causing trauma and without interfering with the incision.

In exemplary embodiments, the medical dressing comprises an absorbent pad between the backing layer and the adhesive skin contact layer.

An absorbent pad is beneficial to absorb the blood arising from the surgical intervention. It is desirable to prevent blood from accumulating at the skin, and instead secure removal and proper handling of the blood and body fluids by means of the absorbent pad. Furthermore, contaminating microorganisms are prevented from accumulating at the skin, and the sterility is thereby improved.

In exemplary embodiments, the absorbent pad comprises a polyurethane foam.

A polyurethane foam is beneficial since it is capable of absorbing large amounts of fluids, and also has a pressure relieving effect. The pressure relieving effect is beneficial to prevent pressure ulcers from occurring. A dressing comprising a polyurethane foam is also conformable.

According to another aspect, there is provided a trocar system comprising a trocar fixation assembly as described hereinbefore and a trocar.

In exemplary embodiments, the trocar comprises a cannula, wherein the cannula comprises an interior surface and an exterior surface, wherein at least a portion of the exterior surface of the cannula is threaded.

The threaded exterior surface of the trocar cannula allows for the trocar cannula to engage with the grooved channel of the ball member. The trocar cannula may be moved along a longitudinal axis of the cannula by means of a screw mechanism. In the absence of a screw mechanism, the trocar cannula is fixed and prevented from projecting in and out of the body cavity.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1A illustrates a trocar fixation assembly according to an exemplary embodiment of the present disclosure associated with a trocar.
Figure 1B illustrates a split view the trocar fixation assembly and the trocar of figure 1A.
Figure 2A illustrates a cut-out view of a trocar fixation assembly of the present disclosure, wherein the ball member is arranged in a straight configuration.
Figure 2B is a zoomed-in, cross-sectional view of the medical dressing of the trocar fixation assembly of figure 2A.
Figure 2C is a zoomed-in view of the trocar fixation assembly of figure 2A.
Figure 2D illustrates a cut-out view of a trocar fixation assembly according to an exemplary embodiment of the present disclosure, wherein the ball member is arranged in a slanted configuration.
Figure 3 illustrates a plurality of trocar fixation assemblies according to exemplary embodiments of the present disclosure when applied to the skin of a patient.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

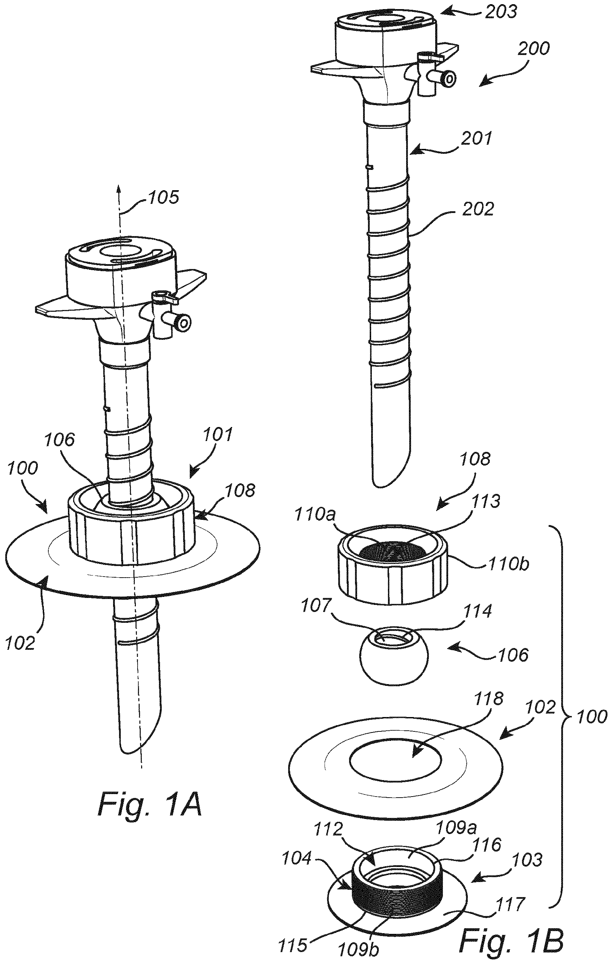

With reference to figures 1A and 1B, a trocar fixation assembly 100 according to an exemplary embodiment of the present disclosure is conceptually illustrated.

The trocar fixation assembly 100 comprises a fixation device 101 and a medical dressing 102, wherein the fixation device 101 comprises:
- a skin attachment element 103 comprising a first annular member 104 disposed about a longitudinal center line 105,
- a ball member 106 arranged in the first annular member 104, wherein the ball member 106 comprises a channel 107 extending through the ball member 106 and being configured to receive a trocar, and
- a second annular member 108 disposed about the longitudinal center line 105 and being configured to engage with the first annular member 104,
wherein the engagement between the first 104 and second 108 annular members allows for the trocar fixation assembly 100 to be shifted between at least a first configuration, in which the ball member 106 is movable with respect to the first 104 and second 108 annular members, a second configuration, in which the ball member 106 is gradually movable with respect to the first 104 and second 108 annular members, and a third configuration, in which the ball member 106 is fixed with respect to the first 104 and second 108 annular members.

As used herein, the term "trocar fixation assembly" means an assembly comprising at least a fixation device and a medical dressing. The medical dressing may be fixedly or detachably attached to the fixation device. The medical dressing is typically arranged to extend around the contour of the fixation device and to be arranged in contact with the skin of the patient. The trocar fixation assembly is to be used in conjunction with a trocar. More particularly, the trocar fixation assembly is used to receive and engage with a trocar cannula.

As used herein, the term "trocar" means a device through which a minimal invasive surgery can be accomplished. The trocar typically comprises at least a cannula; i.e. a hollow tube shaped member, and a gas-tight valve. The trocar allows for the entry of surgical instruments, such as graspers, scissors, staplers, cameras etc.

The term "skin attachment element" means an element adapted to be arranged in contact with the skin of a patient. Typically, the skin attachment element is arranged in an area of the skin surrounding an incision. Accordingly, after an incision has been made in the skin, the skin attachment element is typically arranged to surround the incision and facilitate the positioning and insertion of a trocar.

The "first annular member" is an annular, i.e. ring shaped element defining a first aperture. The first annular member typically forms a closed ring around the first aperture. Accordingly, the first annular member forms a closed path around the first aperture; i.e. it is not openable. The annular member forms part of the skin attachment element. The first annular member is typically formed in one piece. The first annular member is not limited to a particular material but may be formed by any skin compatible or skin friendly material. For example, the first annular member may be formed by a plastic material.

The "ball member" is a ball, or sphere shaped member that comprises a centrally disposed aperture extending through the ball member and defining a channel for receiving a trocar. The ball member is formed in one piece. Accordingly, the parts of the ball member surrounding the channel are formed integrally. The ball member may be formed by any material, but suitably comprises a plastic material. The diameter of the channel of the ball member is adapted to fit with the dimensions of various trocar cannulas.

The "second annular member" is an annular, i.e. ring shaped element defining a second aperture. The second annular member typically forms a closed ring around the second aperture. Accordingly, the second annular member forms a closed path around the second aperture; i.e. it is not openable. The second annular member is typically formed in one piece. The second annular member is not limited to a particular material but may be formed by any skin compatible or skin friendly material. For example, the second annular member may be formed by a plastic material.

The second annular member 108 is "configured to engage" with the first annular member 104; i.e. the second annular member is configured to mate with the first annular member such that the trocar fixation assembly can be shifted between at least a first, a second and a third configuration. The second annular member may be removable from the first annular member. The first and the second annular members are typically configured to engage by means of a threading mechanism. The shifting between the first, second and third configurations may be accomplished by means of a screwing mechanism.

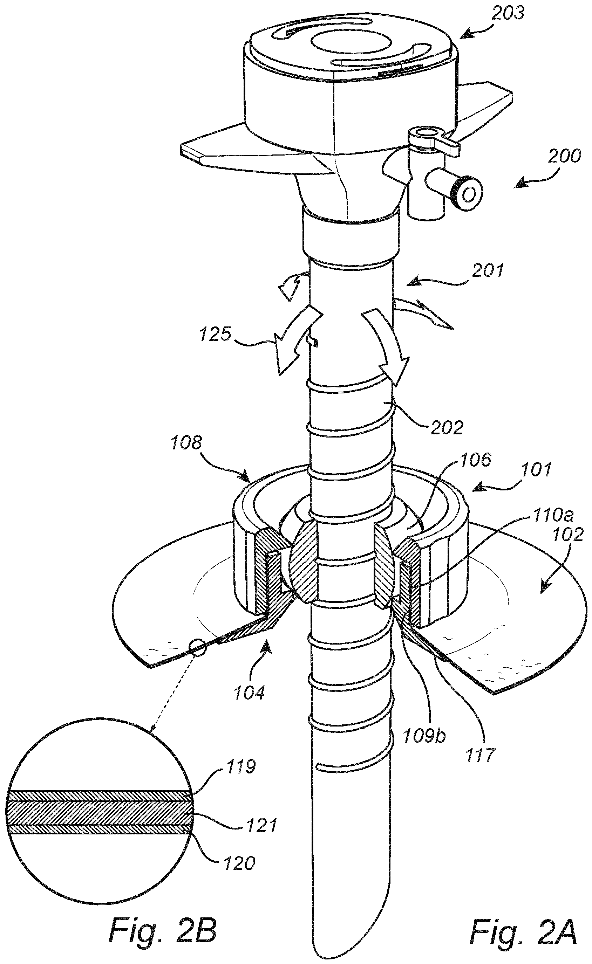

In the first configuration, the ball member 106 is movable with respect to the first 104 and the second 108 annular members. The ball member 106 may be rotated in the circumferential direction with respect to the longitudinal center line 105 (and with respect to the first 104 and/or second 108 annular members) and tilted towards the second annual member 108. This is illustrated by the arrows 125 in figure 2A.

Accordingly, the surgeon may adjust the angular position of the trocar depending on his/her position in the room and depending on the specific surgical situation.

In the second configuration, the ball member is gradually movable with respect to the first 104 and second 108 annular members. Accordingly, the ball member 106 may be moved, but in a more constrained and controllable manner. Undesired rotational movement inside the body cavity is thus prevented. The surgeon or surgical staff may, upon grasping and moving a trocar residing in the trocar fixation assembly, still move the ball member such that a situation-specific adjustment is provided.

In the third configuration, the ball member 106 is fixed with respect to the first 104 and second 108 annular members.

In instances where it is desired for the trocar to be completely fixed in position, the fixation assembly may be shifted to the third configuration. In this configuration, the ball member is prevented from movement in any direction.

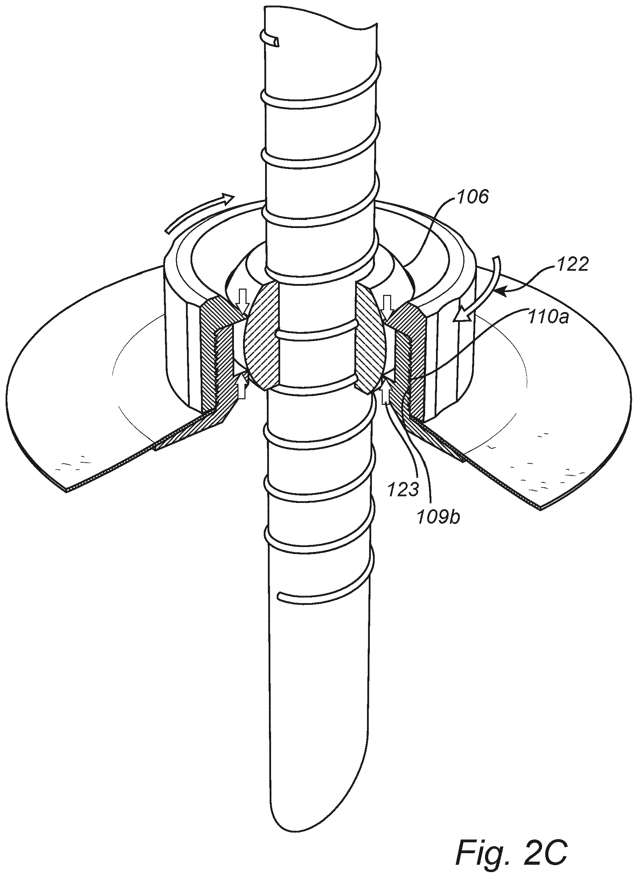

The ball member may be shifted between the first, second and third configuration during the surgical intervention. This may be accomplished by e.g. screwing the second annular member 108 towards the first annular member 104, as illustrated by the arrows 122 in figure 2C.

Upon screwing in the direction of the arrows 122, the interior walls 110a of the second annular member 108 are squeezed against the exterior walls 109b of the first annular member and against the ball member 106. Accordingly, the ball member 106 is fixed in position (illustrated by the arrows 123).

The shifting between the first, second and third configurations of the trocar fixation assembly may be accomplished in a simple and easily adjustable manner.

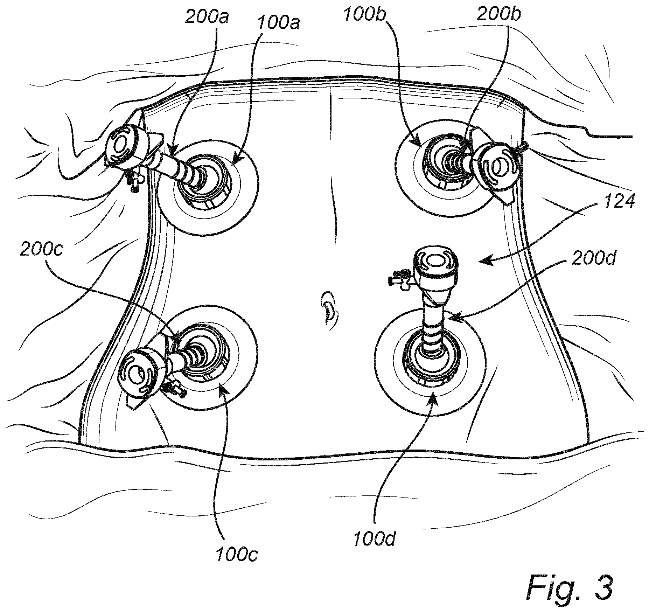

The reversible shifting between the various configurations of the trocar fixation assembly is desirable in MIS procedures involving a plurality of trocars, which each may require a particular position, e.g. angular position, with respect to the body and with respect to the surgeon's position in the room. Such a scenario is schematically illustrated in figure 3, wherein a first 100a, a second 100b, a third 100c, and a fourth 100d trocar fixation assembly are attached to the skin 124 of a patient in a variety of configurations.

The flexibility in shifting the position of the trocars (200a-d), yet being able to fix each trocar (200a-d) in a specific position, greatly facilitates the MIS procedure for the surgical staff, and provides for a safe intervention for the patient.

After the surgery is completed, the trocars (200a-d) may simply and gently be removed, either separately from the trocar fixation assemblies (100a-d), or in conjunction therewith.

Accordingly, the attachment and detachment of the trocar fixation assembly of the present disclosure can be carried out in a fast and simple manner. Compared to conventional means to detach and remove a fixed trocar from a surgical site, a safer solution for the patient is provided. Compared to e.g. balloon trocars and Hasson trocars, which represent common trocars in the field, the attachment and subsequent detachment is significantly improved. A balloon trocar comprises a balloon at the end of the trocar (inserted into the patient to fixate the trocar), which must be deflated prior to removal from the body cavity. In case the step of deflation is missed, risks for the patient may arise. A Hasson trocar typically requires sutures into the skin of a patient in order to fixate the trocar. Before removal of the trocar, the sutures must be removed. With the trocar fixation assembly of the present disclosure, no sutures are required, and no parts remain inside the body cavity.

As best illustrated in figure 1B, each of the first 104 and the second 108 annular members comprises interior walls 109a, 110a and exterior walls 109b, 110b, wherein at least a portion of the exterior walls 109b of the first annular member 104 is threaded, and wherein at least a portion of the interior walls 110b of the second annular member 108 is threaded, wherein the first 104 and second 108 annular members are configured to be threadedly engaged.

The threaded portion of the exterior walls 109b of the first annular member cooperate with the threaded portion of the interior walls 110a of the second annular member and allow for the trocar fixation to be shifted between the first, second and third configurations.

The trocar fixation assembly may be shifted between the first, second and third configurations by means of a screw mechanism.

In this context, "threaded" means that the interior and/or exterior walls of the annular members comprise a plurality of annular ridges extending along the perimeter of at least a portion of the interior and/or exterior walls.

The threaded configuration of the first annular member may be a "female" threading, and the threaded configuration of the second annular member may be a "male threading", or vice versa.

Typically at least 70% of the exterior walls 109b of the first annular member 104 is threaded, e.g at least 80%, e.g. at least 90%.

Typically at least 70% of the interior walls 110a of the second annular member 108 is threaded, e.g at least 80%, e.g. at least 90%.

The ball member 106 is movable or gradually movable in a circumferential direction with respect to the first 104 and/or the second 108 annular member.

A trocar inserted into the channel of the ball member is moveable or gradually moveable in a circumferential direction with respect to the longitudinal center line 105 of the trocar fixation assembly 100.

Accordingly, a trocar may be rotated 360° with respect to the longitudinal center line 105.

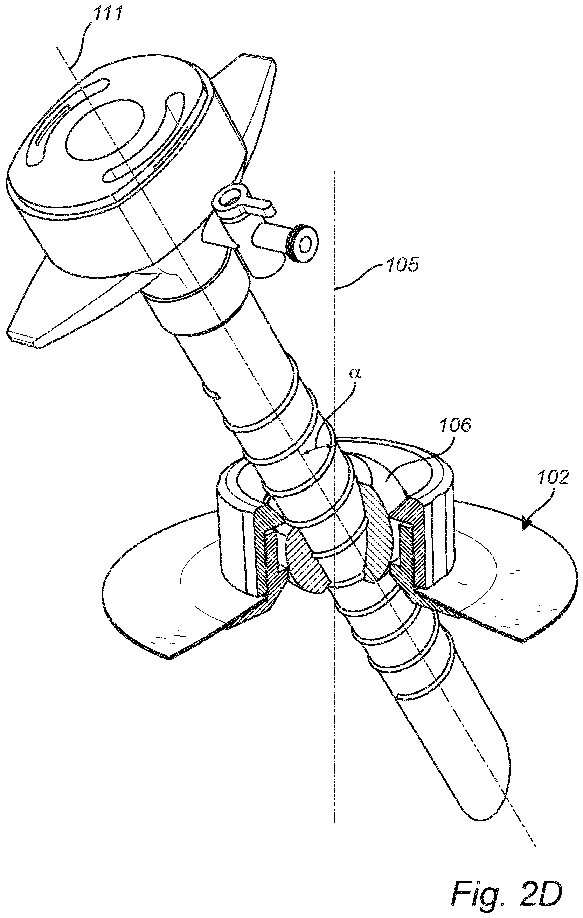

As best illustrated in figure 2D, the ball member 106 is disposed about a longitudinal axis 111, and wherein the ball member 106 may be shifted between a straight configuration, in which the longitudinal axis 111 of the ball member 106 coincides with the longitudinal center line 105, and a slanted configuration, in which the longitudinal axis 111 of the ball member 106 is arranged at an angle, α, with respect to the longitudinal center line 105.

The trocar may e.g. be fixed in a straight configuration in instances where this is desired, and in a slanted configuration, as illustrated in figure 2D, when it is desirable that the trocar is inserted or positioned at an angle, α, with respect to the surgical site. The slanted configuration allows for the surgeon to reach and access the trocar regardless of his/her position in the room.

In embodiments, the longitudinal axis 111 of the ball member 106 is arranged at an angle, α, of from 5 to 60°, preferably from 10 to 45° with respect to the longitudinal center line 105.

The second annular member 108 may restrict the ball member 106 to be tilted such that the angle, α, between the longitudinal axis 111 of the ball member 106 and the longitudinal center line 105 becomes too large. A too large tilting may be harmful to the patient during the surgical intervention.

The channel 107 extending through the ball member 106 is symmetric about the longitudinal axis 111. The longitudinal extension; i.e. the length of the channel may be from 10 to 40 mm, e.g. from 20 to 30 mm.

The ball member 106 is rotatable about the longitudinal axis 111 of the ball member 106, and about the longitudinal center line 105.

The rotatable configuration allows for a trocar to be rotated 360 degrees about the longitudinal center line 105 of the trocar fixation assembly 100 (illustrated by the arrows 125 in figure 2A). The slanted configuration illustrated in figure 2D allows for the surgeon to manipulate, angle and/or fix the trocar 200 in a desired position. Accordingly, the surgical intervention becomes more ergonomic, efficient, and faster.

With reference to figure 1B, the first annular member 104 defines a first aperture 112 having a diameter, d1; the second annular member 108 defining a second aperture 113 having a diameter, d2, wherein the diameter, d3, of the channel 107 of the ball member 106 is smaller than the diameter, d1, of the first aperture 112 and the diameter, d2, of the second aperture 113.

The diameter, d3, of the channel 107 the ball member 106 is adapted to receive and engage with a trocar cannula inserted therethrough. Accordingly, the diameter, d3, may be in the range of from 4 to 15 mm, e.g. from 5 to 12 mm.

The diameter, d1, of the first aperture 112 of the first annular member 104 is not limited to a particular diameter, as long as it allows for the ball member 106 to move freely in the first annular member 104 in the first configuration. For example, the diameter, d1, of the first aperture 112 may be in the range from 20 to 60 mm, e.g. from 30 to 40 mm.

The diameter, d2, of the second aperture 113 of the second annular member 108 is not limited to a particular diameter as long as it enables engagement between the first 104 and the second 108 annular members. Accordingly, the diameter, d2, may be larger than the diameter, d1, of the first aperture 112 of the first annular member 104. For example, the diameter, d3, of the second aperture 113 of the second annular member 108 may be 5 to 15 % larger than the diameter, d1, of the first aperture 112 of the first annular member 104.

As illustrated in figure 1B, at least a portion the channel 107 of the ball member 106 comprises a plurality of annual grooves 114.

This is particularly beneficial when a threaded, e.g. ribbed, trocar cannula is utilized.

The annular grooves 114 may be complementary to the ridges or threads of the exterior surface of a trocar cannula. Accordingly, a trocar may be threadedly engaged with the grooves 114 of the channel and may also be threadedly rotated therein.

The threaded fit between the cannula and the channel 107 allows for the trocar cannula to be fixed in the channel 107. Accordingly, the trocar cannula is prevented from movement in the longitudinal direction; i.e. from projecting into and out of the body cavity during the MIS procedure. Instead, the surgeon may adjust the length of the trocar cannula to be inserted into the body cavity.

The interior walls 109a and exterior walls 109b of the first annular member 104 extend from a bottom surface 115 to a top surface 116, and wherein the skin attachment element 103 comprises a base member 117 surrounding the bottom surface 115 of the first annular member 104.

The base member 117 may be formed integrally with the first annular member 104. Alternatively, the base member 117 may be attached to the first annular member, e.g. by welding. The base member 117 is not limited to a particular shape, but is in figure 1B, circular. The base member 117 of the skin attachment element is the part arranged in contact with the skin.

The base member 117 may be configured to extend radially around the bottom surface 115 of the first annular member 104. The base member 117 has a larger surface area than the surface area of the first annular member 104.

As illustrated in figure 1B, the medical dressing 102 comprises a centrally disposed aperture 118 configured to encircle the first annular member 104.

The diameter of the aperture 118 of the medical dressing 102 substantially corresponds to the diameter, d1, of the first annular member 104.

At least a portion of the medical dressing overlies the base member 117 of the skin attachment element 103.

The medical dressing 102 is illustrated as a circular dressing in the figures. However, the medical dressing is by no means limited to a specific shape, but any shape is conceivable. It is also conceivable that the surgical staff may cut the dressing into a shape specifically adapted for a specific surgical intervention in an actual surgical scenario.

As illustrated in figures 1A and 1B, the medical dressing 102 is arranged between the base member 117 and the second annular member 108. The dressing may be clamped between the skin attachment element 103; i.e. the base member 117, and the second annular member 108.

The medical dressing 102 may be detachably or fixedly attached to the skin attachment element 103. For example, the medical dressing 102 may be detachably or fixedly attached to the base member 117 of the skin attachment element 103

The detachable configuration of the medical dressing 102 allows for each of the components of the trocar fixation assembly to be packed separately such that the surgical staff may assemble the components of the fixation assembly prior to surgery.

If the medical dressing 102 is fixedly attached, the trocar fixation assembly may be packaged as one individual component, which may save time since no assembly step is required prior to surgery.

Preferably, the medical dressing is detachably attached to the fixation device. As illustrated in figures 1-3, the medical dressing 102 extends uninterrupted around the first annular member 104.

Accordingly, the medical dressing 102 forms a closed path around the first annular member 104. Accordingly, no part of the dressing is openable after the dressing has been applied to the skin. This is beneficial to secure a tight seal to the skin and also to prevent contaminants from entering the incision or the area of the skin circumventing the incision.

As illustrated in figure 2B, the medical dressing comprises a backing layer 119 and an adhesive skin contact layer 120.

The backing layer 119 is an outermost layer of the medical dressing and may also be referred to as a top layer. The backing layer 119 faces away from the patient's body in use.

The backing layer 119 may comprise a polymeric film, e.g. a polyethylene film, a polypropylene film or a polyurethane film. Preferably, the backing layer 119 comprises a polyurethane film. The thickness of the backing layer may be in the range of from 10 to 50 µm, e.g. from 15 to 40 µm.

As used herein, the term "adhesive skin contact layer" means a layer configured to detachably adhere the dressing to a dermal surface. In other words, the adhesive skin contact layer is configured to contact the skin of a wearer.

A release liner (not shown) may being co-extensive with the adhesive skin contact layer 120 is typically detachably attached to the adhesive skin contact layer 120. The release liner prevents contamination of the adhesive skin contact layer 120 and is removed prior to application of the dressing to the skin.

The adhesive skin contact layer may comprise any skin-compatible adhesive. Preferably, the adhesive skin contact layer 120 comprises a silicone based adhesive.

A silicone-based adhesive is gentle to the skin and may be removed and applied to the skin in a gentle manner, without causing any trauma. For example, the adhesive skin contact layer 120 may comprise a silicone gel. The silicone gel may be provided as a coating on the backing layer or a pad, if present.

The adhesive skin contact layer 120 may comprise one or more sub-layers. For example, the adhesive skin contact layer 120 may comprise a polymeric film and an adhesive silicone gel layer, wherein the adhesive silicone gel layer is arranged to contact the skin.

The medical dressing further comprises an absorbent pad 121 between the backing layer 119 and the adhesive skin contact layer 120.

The absorbent pad 121 keeps the area circumventing the incision and the trocar fixation assembly free from blood. The absorbent pad 121 absorbs the blood and prevents maceration at the surgical site. Furthermore, the absorbent pad prevents contaminating microorganisms from accumulating at the skin or incision site.

In embodiments where the dressing comprises a pad, the backing layer 119 may be adhesively attached to the pad. Alternatively, the backing layer 119 may be laminated to the pad. For example, heat lamination may be utilized to apply the backing layer 119 to the pad.

The absorbent pad 121 preferably comprises a polyurethane foam.

Typically, the polyurethane foam is a hydrophilic polyurethane foam.

A polyurethane foam has a pressure relieving effect and is capable of absorbing large amounts of fluids

The absorbent pad 121 may comprise one or more layers. If the pad comprises a plurality of pad-forming layers, the pad-forming layers may be laminated or attached to each other.

According to another aspect, there is provided a trocar system comprising a trocar fixation assembly 100 as described hereinbefore and a trocar 200.

The trocar system may be packaged and distributed in a unit such that the surgical staff does not need to assemble the trocar system prior to surgery.

Alternatively, the trocar fixation assembly, and the trocar, respectively, are packaged separately and assembled prior to use.

The trocar 200 comprises a cannula 201. The cannula serves as the "working channel" through which a variety of surgical tools can be inserted. The present disclosure is not limited to a particular cannula, but any cannula may be utilized.

The cannula is typically a hollow tube made of plastic or metal. The cannula may comprise a gas-tight valve 203 that provides for an internal air-seal, allowing instruments to move in and out of the cannula without loss of pneumoperitoneum; i.e. gas present in the body cavity. The valve may be manually or automatically retractable during instrument passage.

The tip of the cannula may be conical, blunt, eccentric or pyramidal to facilitate penetration and entry into a body cavity.

In embodiments, where the channel 107 of the ball member 106 comprises annular grooves 114, a threaded cannula may be beneficial.

The threaded exterior surface of the trocar cannula allows for the trocar cannula 201 to engage with the grooved channel 107 of the ball member 106. The trocar cannula 201 may be moved along the longitudinal axis 111 of the ball member by means of a screw mechanism. In the absence of a screw mechanism, the trocar cannula 201 is fixed and prevented from projecting in and out of the body cavity.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A trocar fixation assembly (100) comprising a fixation device (101) and a medical dressing (102), wherein said fixation device (101) comprises:
- a skin attachment element (103) comprising a first annular member (104) disposed about a longitudinal center line (105),
- a ball member (106) arranged in said first annular member (104), wherein said ball member (106) comprises a channel (107) extending through said ball member (106) and being configured to receive a trocar, and
- a second annular member (108) disposed about said longitudinal center line (105) and being configured to engage with said first annular member (104),
wherein the engagement between said first (104) and second (108) annular members allows for said trocar fixation assembly (100) to be shifted between at least a first configuration, in which said ball member (106) is movable with respect to said first (104) and second (108) annular members, a second configuration, in which said ball member (106) is gradually movable with respect to said first (104) and second (108) annular members, and a third configuration, in which said ball member (106) is fixed with respect to said first (104) and second (108) annular members, wherein each of said first (104) and said second (108) annular members comprises interior walls (109a,1 10a) and exterior walls (109b,1 10b); at least a portion of the exterior walls (109b) of said first annular member (104) being threaded, and at least a portion of the interior walls (110b) of said second annular member (108) being threaded, wherein said first (104) and second (108) annular members are configured to be threadedly engaged, **characterized in that** said medical dressing comprises a centrally disposed aperture (118) configured to encircle said first annular member (104), and wherein said medical dressing (102) comprises a backing layer (119), an adhesive skin contact layer (120) and an absorbent pad (121) between said backing layer (119) and said adhesive skin contact layer (120).

2. The trocar fixation assembly (100) according to claim 1, wherein said ball member (106) is disposed about a longitudinal axis (111), and wherein said ball member (106) may be shifted between a straight configuration, in which the longitudinal axis (111) of said ball member (106) coincides with said longitudinal center line (105), and a slanted configuration, in which the longitudinal axis (111) of said ball member (106) is arranged at an angle, α, with respect to said longitudinal center line (105).

3. The trocar fixation assembly (100) according to claim 2, wherein the longitudinal axis (111) of said ball member (106) is arranged at an angle, α, of from 5 to 60°, preferably from 10 to 45° with respect to said longitudinal center line (105).

4. The trocar fixation assembly (100) according to any one of the preceding claims, wherein said first annular member (104) defines a first aperture (112) having a diameter, d1; said second annular member (108) defining a second aperture (113) having a diameter, d2, wherein the diameter, d3, of said channel (107) of said ball member (106) is smaller than the diameter, d1, of said first aperture (112) and the diameter, d2, of said second aperture (113).

5. The trocar fixation assembly (100) according to any one of the preceding claims, wherein at least a portion of said channel (107) of said ball member (106) comprises a plurality of annual grooves (114).

6. The trocar fixation assembly (100) according to any one of the preceding claims, wherein the interior walls (109a) and exterior walls (109b) of said first annular member (104) extend from a bottom surface (115) to a top surface (116), and wherein said skin attachment element (103) comprises a base member (117) surrounding said bottom surface (115) of said first annular member (104).

7. The trocar fixation assembly (100) according to claim 6, wherein said medical dressing (102) is arranged between said base member (117) and said second annular member (108).

8. The trocar fixation assembly (100) according to any one of the preceding claims, wherein said medical dressing (102) extends uninterrupted around said first annular member (104).

9. The trocar fixation assembly (100) according to any one of the preceding claims, wherein said adhesive skin contact layer (120) comprises a silicone based adhesive.

10. The trocar fixation assembly (100) according to any one of the preceding claims, wherein said absorbent pad (121) comprises a polyurethane foam.

11. A trocar system comprising a trocar fixation assembly (100) according to any one of the preceding claims and a trocar (200).

12. A trocar system according to claim 11, wherein said trocar (200) comprises a cannula (201), wherein said cannula (201) comprises an interior surface and an exterior surface (202), wherein at least a portion of said exterior surface (202) of said cannula (201) is threaded.

## Patentansprüche

1. Trokarfixierungsanordnung (100), die eine Fixierungsvorrichtung (101) und einen medizinischen Verband (102) umfasst, wobei die Fixierungsvorrichtung (101) Folgendes umfasst:
- ein Hautbefestigungselement (103), das ein erstes ringförmiges Element (104) umfasst, das um eine Mittellinie in Längsrichtung (105) angeordnet ist,
- ein Kugelelement (106), das in dem ersten ringförmigen Element (104) angeordnet ist, wobei das Kugelelement (106) einen Kanal (107) umfasst, der durch das Kugelelement (106) verläuft und so ausgelegt ist, dass er einen Trokar aufnimmt, und
- ein zweites ringförmiges Element (108), das um die Mittellinie in Längsrichtung (105) angeordnet ist und so ausgelegt ist, dass es in das erste ringförmige Element (104) eingreift,
wobei sich die Trokarfixierungsanordnung (100) durch die Eingriffbeziehung zwischen dem ersten (104) und zweiten (108) ringförmigen Element zwischen mindestens einer ersten Gestaltung, in der sich das Kugelelement (106) bezogen auf das erste (104) und zweite (108) ringförmige Element bewegen lässt, einer zweiten Gestaltung, in der das Kugelelement (106) bezogen auf das erste (104) und zweite (108) ringförmige Element allmählich bewegen lässt, und einer dritten Gestaltung verstellen lässt, in der das Kugelelement (106) bezogen auf das erste (104) und zweite (108) ringförmige Element fest ist, wobei sowohl das erste (104) als auch das zweite (108) ringförmige Element Innenwände (109a, 110a) und Außenwände (109b, 110b) umfasst; wobei zumindest ein Abschnitt der Außenwände (109b) des ersten ringförmigen Elements (104) ein Gewinde aufweist, und mindestens ein Abschnitt der Innenwände (110b) des zweiten ringförmigen Elements (108) ein Gewinde aufweist, wobei das erste (104) und zweite (108) ringförmige Element so ausgelegt sind, dass sie über eine Schraubverbindung ineinandergreifen, **dadurch gekennzeichnet, dass** der medizinische Verband eine mittig angeordnete Öffnung (118) umfasst, die so ausgelegt ist, dass sie das erste ringförmige Element (104) umschließt, und wobei der medizinische Verband (102) eine Trägerschicht (119), eine haftende Hautkontaktschicht (120) und ein absorbierendes Kissen (121) zwischen der Trägerschicht (119) und der haftenden Hautkontaktschicht (120) umfasst.

2. Trokarfixierungsanordnung (100) nach Anspruch 1, wobei das Kugelelement (106) um eine Längsachse (111) herum angeordnet ist, und wobei das Kugelelement (106) zwischen einer geraden Gestaltung, in der die Längsachse (111) des Kugelelements (106) mit der Mittellinie in Längsrichtung (105) übereinstimmt, und einer schrägen Gestaltung verstellt werden kann, in der die Längsachse (111) des Kugelelements (106) bezogen auf die Mittellinie in Längsrichtung (105) unter einem Winkel, α, angeordnet ist.

3. Trokarfixierungsanordnung (100) nach Anspruch 2, wobei die Längsachse (111) des Kugelelements (106) bezogen auf die Mittellinie in Längsrichtung (105) unter einem Winkel, α, von 5 bis 60°, vorzugsweise von 10 bis 45° angeordnet ist.

4. Trokarfixierungsanordnung (100) nach einem der vorhergehenden Ansprüche, wobei das erste ringförmige Element (104) eine erste Öffnung (112) mit einem Durchmesser, d1, definiert; wobei das zweite ringförmige Element (108) eine zweite Öffnung (113) mit einem Durchmesser, d2, definiert, wobei der Durchmesser, d3, des Kanals (107) des Kugelelements (106) kleiner ist als der Durchmesser, d1, der ersten Öffnung (112) und der Durchmesser, d2, der zweiten Öffnung (113).

5. Trokarfixierungsanordnung (100) nach einem der vorhergehenden Ansprüche, wobei zumindest ein Abschnitt des Kanals (107) des Kugelelements (106) eine Vielzahl von jährlichen Rillen (114) umfasst.

6. Trokarfixierungsanordnung (100) nach einem der vorhergehenden Ansprüche, wobei die Innenwände (109a) und Außenwände (109b) des ersten ringförmigen Elements (104) von einer unteren Fläche (115) zu einer oberen Fläche (116) verlaufen und wobei das Hautbefestigungselement (103) ein Bodenelement (117) umfasst, das die untere Fläche (115) des ersten ringförmigen Elements (104) umgibt.

7. Trokarfixierungsanordnung (100) nach Anspruch 6, wobei der medizinische Verband (102) zwischen dem Bodenelement (117) und dem zweiten ringförmigen Element (108) angeordnet ist.

8. Trokarfixierungsanordnung (100) nach einem der vorhergehenden Ansprüche, wobei der medizinische Verband (102) ununterbrochen um das ringförmige Element (104) herum verläuft.

9. Trokarfixierungsanordnung (100) nach einem der vorhergehenden Ansprüche, wobei die haftende Hautkontaktschicht (120) einen Klebstoff auf Silikonbasis umfasst.

10. Trokarfixierungsanordnung (100) nach einem der vorhergehenden Ansprüche, wobei das absorbierende Kissen (121) einen Polyurethanschaum umfasst.

11. Trokarsystem, das eine Trokarfixierungsanordnung (100) nach einem der vorhergehenden Ansprüche und ein Trokar (200) umfasst.

12. Trokarsystem nach Anspruch 11, wobei der Trokar (200) eine Kanüle (201) umfasst, wobei die Kanüle (201) eine Innenfläche und eine Außenfläche (202) umfasst, wobei zumindest ein Abschnitt der Außenfläche (202) der Kanüle (201) ein Gewinde aufweist.

## Revendications

1. Ensemble de fixation de trocart (100) comprenant un dispositif de fixation (101) et un pansement médical (102), ledit dispositif de fixation (101) comprenant :
- un élément de fixation à la peau (103) comprenant un premier élément annulaire (104) disposé autour d'une ligne centrale longitudinale (105),
- un élément à bille (106) disposé dans ledit premier élément annulaire (104), ledit élément à bille (106) comprenant un canal (107) s'étendant à travers ledit élément à bille (106) et qui est configuré pour recevoir un trocart, et
- un second élément annulaire (108) disposé autour de ladite ligne centrale longitudinale (105) et qui est configuré pour s'engager dans ledit premier élément annulaire (104),
l'engagement entre lesdits premier (104) et second (108) éléments annulaires permettant audit ensemble de fixation de trocart (100) d'être décalé entre au moins une première configuration dans laquelle l'élément à bille (106) est mobile par rapport auxdits premier (104) et second (108) éléments annulaires, une deuxième configuration dans laquelle ledit élément à bille (106) est mobile graduellement par rapport auxdits premier (104) et second (108) éléments annulaires, et une troisième configuration dans laquelle ledit élément à bille (106) est fixe par rapport auxdits premier (104) et second (108) éléments annulaires, chacun desdits premier (104) et second (108) éléments annulaires comprenant des parois intérieures (109a, 110a) et des parois extérieures (109b, 110b), au moins une section des parois extérieures (109b) dudit premier élément annulaire (104) étant filetée, et au moins une section des parois intérieures (110b) dudit second élément annulaire (108) étant filetée, lesdits premier (104) et second (108) éléments annulaires étant configurés pour s'engager par filetage, **caractérisé en ce que** ledit pansement médical comprend une ouverture pratiquée au centre (118) et configurée pour encercler le premier élément annulaire (104), et ledit pansement médical (102) comprenant une couche support (119), une couche adhésive en contact avec la peau (120) et un tampon absorbant (121) entre ladite couche support (119) et ladite couche adhésive en contact avec la peau (120).

2. Ensemble de fixation de trocart (100) selon la revendication 1, dans lequel ledit élément à bille (106) est disposé autour d'un axe longitudinal (111) et ledit élément à bille (106) peut être décalé entre une configuration droite, dans laquelle l'axe longitudinal (111) dudit élément à bille (106) coïncide avec ladite ligne centrale longitudinale (105), et une configuration inclinée, dans laquelle l'axe longitudinal (111) dudit élément à bille (106) est disposé suivant un angle α par rapport à ladite ligne centrale longitudinale (105).

3. Ensemble de fixation de trocart (100) selon la revendication 2, dans lequel l'axe longitudinal (111) dudit élément à bille (106) est disposé suivant un angle α de 5 à 60°, de préférence de 10 à 45°, par rapport ladite ligne centrale longitudinale (105).

4. Ensemble de fixation de trocart (100) selon l'une quelconque des revendications précédentes, dans lequel ledit premier élément annulaire (104) définit une première ouverture (112) ayant un diamètre d1 ; ledit second élément annulaire (108) définit une seconde ouverture (113) ayant un diamètre d2, le diamètre d3 dudit canal (107) dudit élément à bille (106) étant inférieur au diamètre d1 de ladite première ouverture (112) et au diamètre d2 de ladite seconde ouverture (113) .

5. Ensemble de fixation de trocart (100) selon l'une quelconque des revendications précédentes, dans lequel au moins une partie dudit canal (107) dudit élément à bille (106) comprend une pluralité de gorges annulaires (114) .

6. Ensemble de fixation de trocart (100) selon l'une quelconque des revendications précédentes, dans lequel les parois intérieures (109a) et les parois extérieures (109b) dudit premier élément annulaire (104) s'étendent depuis une surface inférieure (115) jusqu'à une surface supérieure (116), et ledit élément de fixation à la peau (103) comprend un élément de base (117) entourant ladite surface inférieure (115) dudit premier élément annulaire (104).

7. Ensemble de fixation de trocart (100) selon la revendication 6, dans lequel ledit pansement médical (102) est disposé entre ledit élément de base (117) et ledit second élément annulaire (108).

8. Ensemble de fixation de trocart (100) selon l'une quelconque des revendications précédentes, dans lequel ledit pansement médical (102) s'étend sans interruption autour dudit premier élément annulaire (104).

9. Ensemble de fixation de trocart (100) selon l'une quelconque des revendications précédentes, dans lequel ladite couche adhésive en contact avec la peau (120) comprend un adhésif à base de silicone.

10. Ensemble de fixation de trocart (100) selon l'une quelconque des revendications précédentes, dans lequel ledit tampon absorbant (121) comprend une mousse de polyuréthane.

11. Système de trocart comprenant un ensemble de fixation de trocart (100) selon l'une quelconque des revendications précédentes et un trocart (200).

12. Système de trocart selon la revendication 11, ledit trocart (200) comprenant une canule (201), ladite canule (201) comprenant une surface intérieure et une surface extérieure (202), au moins une partie de ladite surface extérieure (202) de ladite canule (201) étant filetée.
